(19) Europäisches Patentamt
European Patent Office
Office européen des brevets

(11) **EP 1 461 039 B1**

(12) **EUROPEAN PATENT SPECIFICATION**

(45) Date of publication and mention
of the grant of the patent:
**22.10.2014 Bulletin 2014/43**

(51) Int Cl.:
*A61K 31/4709* [(2006.01)]    *A61K 31/522* [(2006.01)]
*A61K 31/519* [(2006.01)]    *A61K 31/4745* [(2006.01)]
*A61P 7/02* [(2006.01)]    *A61P 9/08* [(2006.01)]

(21) Application number: **02796079.8**

(22) Date of filing: **26.12.2002**

(86) International application number:
**PCT/US2002/041531**

(87) International publication number:
**WO 2003/057222 (17.07.2003 Gazette 2003/29)**

(54) **PHARMACEUTICAL COMPOSITIONS COMPRISING CILOSTAZOL AND AN ADENOSINE UPTAKE INHIBITOR**

PHARMAZEUTISCHE ZUBEREITUNGEN ENTHALTEND CILOSTAZOL UND EINEN ADENOSINAUFNAHME-INHIBITOR

COMPOSITIONS PHARMACEUTIQUES COMPORTANT CILOSTAZOL ET UN INHIBITEUR D'APPORT D'ADENOSINE

(84) Designated Contracting States:
**AT BE BG CH CY CZ DE DK EE ES FI FR GB GR IE IT LI LU MC NL PT SE SI SK TR**

(30) Priority: **27.12.2001 US 342367 P**
           **23.09.2002 US 412546 P**

(43) Date of publication of application:
**29.09.2004 Bulletin 2004/40**

(73) Proprietor: **OTSUKA PHARMACEUTICAL CO., LTD.**
**Chiyoda-ku,**
**Tokyo 101-8535 (JP)**

(72) Inventors:
• **LIU, Yongge**
**Germantown, MD 20876 (US)**
• **SUN, Bing**
**Gaithersburg, MD 20878 (US)**
• **YOSHITAKE, Masuhiro**
**Potomac, MD 20854 (US)**
• **KAMBAYASHI, Jun-Ichi**
**Potomac, MD 20854 (US)**

(74) Representative: **Dörries, Hans Ulrich**
**df-mp Dörries Frank-Molnia & Pohlman**
**Patentanwälte Rechtsanwälte PartG mbB**
**Theatinerstrasse 16**
**80333 München (DE)**

(56) References cited:
**US-A1- 2002 032 171      US-A1- 2002 147 184**

• **LIU ET AL.: "Inhibition of Adenosine Uptake and Augmentation of Ischemia-Induced Increase of Interstitial Adenosine by Cilostazol, an Agent to Treat Intermittent Claudication" JOURNAL OF CARDIOVASCULAR PHARMACOLOGY, vol. 36, 2000, pages 351-360, XP009009579 usa cited in the application**
• **JACKSON, M., R. ET AL.: "Antithrombotic Therapy in Peripheral Arterial Occlusive Disease" CHEST, vol. 119, no. 1 suppl., January 2001 (2001-01), pages 283s-299s, XP002239103**
• **DAWSON, D., L.: "Comparative Effects of Cilostazol and other Therapies for Intermittent Claudication" AMERICAN OURNAL OF CARDIOLOGY, vol. 87, no. 12a, 28 June 2001 (2001-06-28), pages 19d-27d, XP002239104 usa**

**Description**

**FIELD OF THE INVENTION**

The present invention relates to pharmaceutical compositions comprising cilostazol which is a multifunctional phosphodiesterase inhibitor (MPDEI) and at least one adenosine uptake inhibitor. A MPDEI is an agent that, at a minimum, inhibits both phosphodiesterase type III (PDE3) nd adenosine uptake (e.g., cilostazol). The invention also relates to the use of the compositions for treating a variety of symptoms and illnesses including limb ischemia and intermittent claudication (IC) associated with peripheral arterial occlusive disease (PAOD), for the prevention and treatment of stroke, and for the prevention of coronary thrombosis and restenosis. The invention provides the use of the compositions to achieve enhanced therapeutic potency and efficacy with less side effects than those that may occur using either MPDEIs, traditional PDE3 inhibitors, or adenosine uptake inhibitors alone. The ability of the compositions to enhance the antiplatelet and vasodilatory effects, and to circumvent potential cardiotonic side effects of MPDEIs or PDE3 inhibitors, offers the possibility of extending the approved indication and usage of cilostazol to patients that present with IC, stroke, or coronary disease and congestive heart failure (CHF).

**BACKGROUND**

PAOD affects up to 5% of elderly patients in the United States (US), and patients with PAOD have a six-fold increased risk of death from cardiac and cerebrovascular causes. IC is a frequently disabling symptom of PAOD. Patients typically describe discomfort, variably characterized as pain, ache or feeling of fatigue, in the affected leg when walking. There are only two approved drugs in the US for the treatment of IC. Pentoxifylline has been available for two decades but it is only marginally efficacious. Cilostazol (Pletal®) (6-[4-(1-cyclohexyl-1*H*-tetrazol-5yl) butoxy]-3,4-dihydro-2(1*H*)-quinolinone) was approved by the US Food and Drug Administration (FDA) in 1999 for the treatment of IC. In placebo-controlled trials, cilostazol significantly improved maximal walking distance on a treadmill compared with placebo and pentoxifylline.

Cilostazol has long been known as a cyclic nucleotide PDE3 inhibitor. Cyclic nucleotides, such as cyclic adenosine monophosphate (cAMP) and cyclic guanosine monophosphate (cGMP), play an important role in mediating many cellular responses within the cardiovascular system. Intracellular levels of cyclic nucleotides are controlled by the balanced activities of two families of enzymes. Adenylate cyclase and guanylate cyclase regulate the *de novo* synthesis of cAMP and cGMP, respectively. Conversely, eleven genetically distinct isoforms of PDE, which differ in their biochemical and pharmacological profiles, regulate the degradation of cAMP and/or cGMP. The PDE3 isoform acts specifically on cAMP and causes depletion of intracellular cAMP. PDE3 is expressed in a number of different cell types including cardiomyocytes, vascular smooth muscle cells (VSMC), and platelets. Accordingly, PDE3 affects cardiac contractility, VSMC tone and proliferation, and platelet activity, respectively. Inhibition of PDE3 causes a selective accumulation of intracellular cAMP, and an increase in protein kinase A (PKA)-induced effects. Therefore, decreased PDE3 activity in the above cells causes increased cardiac contractility, vasodilation, decreased cellular proliferation, and decreased platelet aggregation. The beneficial effects of cilostazol in patients with IC have been largely attributed to the vasodilatory and anti-platelet aggregation effects of PDE3 inhibition, although other effects may also play a role.

PDE3 inhibitors generally exert positive inotropic and chronotropic effects on the heart (i.e., increased contractility and heart rate). Indeed, PDE3 inhibitors have been shown to increase cardiac output and to reduce pulmonary congestion in patients with CHF. For example, milrinone, a prototypic PDE3 inhibitor, is currently in clinical use for the acute treatment of CHF. However, chronic use of milrinone in patients with CHF has been associated with proarrhythmic activities (probably due to excessive increases of cAMP-induced cardiac contractility (Packer, 1992; Thadani and Roden, 1998)). Cilostazol has not been shown to increase cardiovascular mortality in IC clinical trials in the US, and safe long-term use has been demonstrated in Asian countries (NDA of Cilostazol, Otsuka America Pharmaceutical, Inc., 1997). In general, CHF patients did not participate in the cilostazol IC trials in the US because exercise-limiting CHF was an exclusionary criterion. Thus, relatively few patients with CHF (and none with severe CHF) participated in the clinical trials in the US, and the drug's effect on mortality in this group of patients is unknown. Nevertheless, based on prior clinical experience with PDE3 inhibitors such as milrinone, the FDA has mandated that cilostazol be contraindicated in patients with CHF of any severity. Unfortunately, the population of patients with IC may overlap that with CHF such that the beneficial effects of PDE3 inhibition are not generally available to these patients. Therefore, it is important to develop new pharmacologic approaches that eliminate or minimize the potential cardiac side effects of cilostazol and, thereby, allow the benefits of cilostazol therapy to be extended to patients that exhibit IC and cardiac dysfunction.

**SUMMARY OF THE INVENTION**

The present invention addresses these needs by providing pharmaceutical compositions that inhibit PDE3 activity and adenosine uptake. These pharmaceutical compositions include a combination of cilostazol and at least one adenosine uptake inhibitor (e.g., dipyridamole). In the present invention, the combination of cilostazol and at least one adenosine uptake inhibitor acts synergistically to increase antiplatelet effect and vasodilation, while limiting the positive inotropic effect of PDE3 inhibition. The combination of cilostazol and at least one adenosine uptake inhibitor should be safer and more efficacious than either agent alone for the treatment of a variety of symptoms and illnesses including PAOD (such as IC), stroke, and coronary thrombosis and restenosis.

## BRIEF DESCRIPTION OF THE DRAWINGS

Figure 1 illustrates the synergistic effect of adenosine (1 $\mu$M) and cilostazol (1 $\mu$M) on collagen-induced platelet aggregation. Washed platelets were activated with collagen (1 $\mu$g/ml) as indicated by the arrow.

Figure 2 illustrates the dose-dependent synergistic effect of dipyridamole (1, 3 and 10 $\mu$M) and cilostazol (10 pM to 100 $\mu$M) on platelet aggregation in washed platelets.

Figure 3 illustrates the synergistic effect of dipyridamole (1, 3 and 10 $\mu$M) on platelet aggregation in washed platelets in the presence of adenosine (1$\mu$M) and cilostazol (30 nM and 100 nM).

Figure 4 illustrates the synergistic effect of cilostazol (30 and 100 nM) on platelet aggregation in washed platelets in the presence of adenosine (1$\mu$M) and dipyridamole (1, 3 and 10 $\mu$M).

Figure 5 illustrates the synergistic effect of dipyridamole (3 $\mu$M) and cilostazol (3 $\mu$M) on intracellular cAMP level elevation in the platelets of PRP, in the presence of adenosine (0.3 $\mu$M and 1 $\mu$M).

Figure 6 illustrates the synergistic effect of dipyridamole (0.5, 1, 5 and 10 $\mu$M) and cilostazol (1 $\mu$M) on intracellular cAMP level elevation in adenosine $A_{2A}$-expressing Chinese hamster ovary (CHO) cells, in the presence or absence of adenosine (1 $\mu$M).

Figure 7 illustrates the synergistic effect of dipyridamole (0.5, 1, 5 and 10 $\mu$M) and cilostazol (3 $\mu$M) on intracellular cAMP level elevation in adenosine $A_{2A}$-expressing Chinese hamster ovary (CHO) cells, in the presence or absence of adenosine (1 $\mu$M).

Figure 8 illustrates the inhibitory effect of cilostazol in comparison with milrinone on adenosine uptake into washed human platelets and erythrocytes.

Figure 9 illustrates the increase in adenosine levels in plasma with collagen (2 $\mu$g/ml) stimulation in the presence or absence of dipyridamole (1 $\mu$M) in whole blood.

Figure 10 illustrates the synergistic effect of dipyridamole (0.1, 0.3, 1 and 3 $\mu$M) and cilostazol (10 $\mu$M and 30 $\mu$M) on platelet aggregation in whole blood induced by 0.5 $\mu$g/ml of collagen.

Figure 11 illustrates the synergistic effect of dipyridamole (1 and 3 $\mu$M) and low concentrations of cilostazol (0.3, 0.7, 1, and 3 $\mu$M) on whole-blood platelet aggregation induced by 0.1 or 0.3 $\mu$g/ml of collagen.

Figure 12 illustrates the synergistic effect of dipyridamole (~1 $\mu$M) and cilostazol (~1 $\mu$M) on the inhibition of whole blood platelet aggregation *ex vivo.*

Figure 13 illustrates the experimental protocols used to study the effect of cilostazol and dipyridamole on cardiac function of isolated rabbit Langendorff hearts.

Figure 14 illustrates the effect of cilostazol (1, 3, and 10 $\mu$M) and dipyridamole (0.3, 1 and 3 $\mu$M) alone or in combination on contractility (A), heart rate (B), and coronary flow (C).

Figure 15 illustrates the protocol for testing the effect of the combination of low levels of cilostazol and dipyridamole on gastrocnemius muscle blood flow during rest, exercise (with electric stimulation), and ischemia by occluding the femoral artery and reperfusion.

Figure 16 illustrates that treatment with the combination of cilostazol (1 $\mu$M) and dipyridamole (1 $\mu$M) significantly increased blood flow in the exercised gastrocnemius muscle, and improved blood flow recovery after a period of ischemia compared to those in the untreated muscle.

## DETAILED DESCRIPTION OF THE INVENTION

The present invention addresses the need in the art for safe and effective pharmaceutical compositions for such conditions as PAOD (such as IC), stroke, coronary thrombosis, and restenosis. Cilostazol is available for the treatment of IC and has been shown to be effective in the prevention of stroke (Gotoh, Tohgi, Hirai, Terashi, Fukuuchi, Otomo, Shinohara, Itoh, Matsuda, Sawada, Yamaguchi, Nishimaru, and Ohashi, 2000), coronary thrombosis after coronary percutaneous transluminal coronary angioplasty (PTCA) (Park, Lee, Kim, Lee, Park, Hong, Kim, and Park, 1999) and restenosis (Tsuchikane, Fukuhara, Kobayashi, Kirino, Yamasaki, Izumi, Otsuji, Tateyama, Sakurai, and Awata, 1999). Cilostazol inhibits PDE3, and the resultant anti-platelet and vasodilatory effects appear to contribute to its therapeutic action. However, the possible cardiac side effects of PDE3 inhibition are a concern. Indeed, because of prior clinical experiences with milrinone, cilostazol is contraindicated in CHF of any severity.

Recent studies indicate that cilostazol possesses an unexpected mechanism of action that is not shared with milrinone. In fact, cilostazol has been shown to inhibit adenosine uptake into various cells including ventricular myocytes, coronary smooth muscle cells, endothelial cells, erythrocytes, and platelets. Cilostazol inhibits adenosine uptake with an $IC_{50}$ of around 5-10 $\mu$M. In contrast, milrinone has no significant inhibitory effect at concentrations as high as 100 $\mu$M (Liu et al., 2000). Because of its abilities to inhibit PDE3 and adenosine uptake, the inventors consider cilostazol a MPDEI.

Inhibition of adenosine uptake is significant because adenosine induces a wide range of biologic effects including vasodilation and inhibition of platelet aggregation. Adenosine also exerts negative inotropic and chronotropic effects on the heart. The effects of adenosine on the vasculature and platelets are mediated by the activation of adenosine $A_2$ receptors. Adenosine $A_2$ receptors trigger $G_s$ protein to stimulate adenylate cyclase and, thereby, increase the intracellular concentration of cAMP. The well-known anti-adrenergic effects of adenosine on the myocardium are mediated by the activation of adenosine $A_1$ receptors. Adenosine $A_1$ receptors trigger $G_i$ protein to inhibit adenylate cyclase and, thereby, decrease the intracellular concentration of cAMP (Dobson and Fenton, 1998; George et al., 1991; Narayan et al., 2000). Inhibition of adenosine uptake increases interstitial and circulatory levels of adenosine. An increase in extracellular adenosine has the favorable consequences of enhancing the anti-platelet (Sun et al., 2001) and vasodilatory effects of PDE3 inhibition and diminishing the positive inotropic effect of PDE3 inhibition (Wang et al., 2001). The potential antagonistic effect of adenosine on the positive inotropy caused by inhibition of PDE3 was demonstrated by the induction of a smaller increase in cardiac contractility by cilostazol compared with milrinone (Cone et al., 1999), and by the ability of an adenosine $A_1$ antagonist to increase the cardiotonic effect of cilostazol in isolated rabbit hearts (Wang, Cone, Fong, Yoshitake, Kambayashi, and Liu, 2001). In addition, adenosine has been implicated as an important local mediator of the cardioprotection (Downey et al., 1994), and has been shown to attenuate injuries from ischemia and reperfusion in skeletal muscle and neurons (Wang et al., 1996; Whetzel et al., 1997). Overall, adenosine may play a role in the increase in claudication distance brought about by exercise training and may exert a favorable effect on IC-related symptoms (Laghi et al., 1997; Pasini et al., 2000).

The potency of cilostazol for inhibition of adenosine uptake is more than one order of magnitude lower than it is for inhibition of PDE3 (5-10 $\mu$M vs. 0.32 $\mu$M) (Liu et al., 2000). The increase of extracellular adenosine caused by cilostazol, while being sufficient to attenuate positive inotropy and augment anti-platelet aggregation, is mild compared with that caused by the potent adenosine uptake inhibitor dipyridamole ($IC_{50}$ 10 nM). Therefore, one therapeutic approach to increasing the efficacy and decreasing the potential cardiac side effects of cilostazol in the treatment of IC is to combine this MPDEI with at least one adenosine uptake inhibitor (e.g., dipyridamole). The Applicants have discovered that the combination of cilostazol and a potent adenosine uptake inhibitor yields anti-platelet effects greater than those which can be attributed to the additive effect of a PDE3 inhibitor or an adenosine uptake inhibitor alone. Indeed, the combination produced a synergistic inhibition of platelet function confirming the contribution of distinct mechanisms of action. Moreover, the combination has been found to reduce the positive inotropic effects of cilostazol alone. In addition, the combination of low levels of cilostazol and dipyridamole increases blood flow in the exercised gastrocnemius muscle and improves the tissue flow recovery after a period of ischemia (whereas, each drug alone does not change blood flow, significantly). Thus, the resulting combination provides a safe and effective treatment for illnesses involving platelet aggregation and vasoconstriction. These illnesses include PAOD (such as IC), stroke, and coronary thrombosis. This combination can also be used to treat coronary restenosis due to the inhibition of smooth muscle proliferation by cilostazol.

In addition to its beneficial action in IC, cilostazol has been shown to be effective in the prevention of stroke recurrence (Gotoh et al., 2000). While it is not known whether dipyridamole alone is effective, dipyridamole in combination with aspirin is currently marketed as Aggrenox® (Boehringer Ingelheim) for the prevention of stroke. The beneficial effect of Aggrenox® is attributed to the additive anti-platelet effects of dipyridamole and aspirin. Various studies have demonstrated advantages of cilostazol over other anti-platelet agents such as aspirin (Igawa et al., 1990; Matsumoto et al., 1999). Because cilostazol and dipyridamole synergistically inhibit platelet aggregation, the combination of these two drugs should be at least as efficacious in the prevention of stroke.

Cilostazol has been successfully used in the prevention of thrombosis after coronary PTCA (Park et al., 1999), and for the prevention of restenosis after PTCA with or without stent (Tsuchikane et al., 1999). The combination of cilostazol and dipyridamole should be as efficacious and safer than cilostazol alone by reducing the deleterious cardiac side effect.

In one embodiment of the invention, the composition comprises cilostazol and at least one adenosine uptake inhibitor in an amount capable of providing synergistic inhibition of platelet aggregation Another embodiment of the present invention provides compositions comprising cilostazol and at least one adenosine uptake inhibitor in amounts capable of providing synergistic elevation of intracellular cAMP levels. The invention also provides a use for treating PAOD (such as IC), stroke, and coronary thrombosis and restenosis with the compositions to achieve enhanced therapeutic potency and efficacy with less side effects than may occur during treatment with either a PDE3 inhibitor or an adenosine uptake inhibitor alone.

"PDE3 inhibitor" as used herein refers to an agent that is capable of inhibiting or selectively reducing the activity of PDE type III. PDE3 inhibitor according to the invention may be any known or yet to be discovered compound that inhibits PDE3. Acceptable PDE3 inhibitors include the following: bipyridines such as milrinone and amrinone; imidazolones such as piroximone and enoximone; imidazolines such as imazodan and 5-methyl-imazodan; dihydropyridazinones such as indolidan and LY181512; dihydroquinolinone compounds such as cilostamide, cilostazol and OPC 3911; and other compounds such as anagrelide, bemoradan, ibudilast, isomazole, lixazinone, motapizone, olprinone, phthalazinol, pimobendan, quazinone, siguazodan, and trequinsin.

"MPDEI" as used herein refers to an agent that is capable of inhibiting or selectively reducing the activity of PDE3 and is efficacious in blocking adenosine transport into a cell. The MPDEI according to the invention is cilostazol

"Adenosine uptake inhibitor" as used herein refers to any agent which is efficacious in blocking adenosine transport into a cell. Such adenosine uptake inhibitors include those known compounds which have been shown to inhibit adenosine transport. Acceptable adenosine uptake inhibitors according to the invention are the following: dipyridamole; propentofylline; dilazep; nitrobenzylthioinosine; S-(4-nitrobenzyl)-6-thioguanosine; S-(4-nitrobenzyl)-6-thioinosine; iodohydroxy-nitrobenzylthioinosine; mioflazine; pharmaceutically acceptable salts thereof.

The present invention relates to the use of a combination of cilostazol and at least one adenosine up-take inhibitor (i.e., the present pharmaceutical composition) for the treatment of PAOD (such as IC), stroke, coronary thrombosis or other symptoms or illnesses characterized as resulting from excessive platelet aggregation, or arterial occlusion, etc., and coronary restenosis resulting from smooth muscle proliferation. As used herein, pharmaceutically effective refers to an amount of an agent that is able to reduce the rate of occurrence or severity of any of the symptoms or illnesses described above. As is known by those of ordinary skill in this art, symptoms of the above include discomfort or pain in affected limbs, and gangrene, etc. Overall, an efficacious dosage of the pharmaceutical composition will cause reduction of PDE3 activity and adenosine uptake in platelets and other blood cells, as well as vascular smooth muscle cells, in amounts sufficient to prevent, ameliorate, or otherwise treat the symptoms and illnesses described.

Persons of ordinary skill in the art would be able to determine and optimize the dosages of cilostazol and adenosine uptake inhibitors of the instant invention using techniques that are known in the art. Those techniques are set out, for example, on pages 3-41 of Goodman and Gilman's The Pharmacological Basis of Therapeutics, Ninth Edition. (1996) Dosages can be ascertained and optimized through the use of established assays, conventional dose- and time-response studies, and conventional pharmacokinetic and metabolism studies. Further refinements of the calculations necessary to determine the appropriate dosages for treatment are routinely made by those of ordinary skill in the art and are within the array of tasks routinely performed by them without undue experimentation. For example, the data obtained from cell culture assays and animal studies can be used in formulating a range of dosage for use in a patient. The dosage can vary within this range depending upon the dosage form employed and the route of administration utilized. For any composition for use in therapy of the invention, the therapeutically effective dose can be estimated initially from cell culture assays. A dose may be formulated to achieve a circulating blood plasma concentration range as determined in cell culture. When two or more compounds are to be administered, either as a single formulation or as separate formulations, the dose(s) may be formulated to achieve a molar ratio range between the two or more compounds in the circulating blood plasma as determined in cell culture. For example, in one embodiment of the present invention, a composition comprising cilostazol and dipyridamole produces a blood concentration of about 0.3 $\mu$M to about 10 $\mu$M for cilostazol and about 0.1 $\mu$M to about 3 $\mu$M for dipyridamole. In other embodiments, a composition comprising cilostazol and dipyridamole produces a blood concentration of 0.5 $\mu$M to 5 $\mu$M, or 1 $\mu$M to 3 $\mu$M, for cilostazol and 1 $\mu$M to 3 $\mu$M for dipyridamole. Accordingly, in one embodiment of the present invention, a composition comprising cilostazol and dipyridamole produces a cilostazol:dipyridamole molar ratio in blood of about 0.1:1 to about 1:0.01. In other embodiments of the present invention, a composition comprising cilostazol and dipyridamole produces a cilostazol:dipyridamole molar ratio in blood of about 0.16:1 to about 1:0.2, or about 0.33:1 to about 1:0.33. With the current clinically available formulation, these levels of blood concentrations are equivalent to a daily dose of 20 mg to 300 mg for cilostazol (Pletal®) and 200 mg to 600 mg for dipyridamole (Persantine-Retard®). In other embodiments, these levels of blood concentrations are equivalent to a daily dose of 50 mg to 200 mg, or 50 mg to 160 mg, for cilostazol and 200 to 600 mg for dipyridamole. Accordingly, a pharmaceutical preparation comprising cilostazol and dipyridamole has a cilostazol:dipyridamole weight ratio of about 1:0.7 to about 1:30. In other embodiments, a pharmaceutical preparation comprising cilostazol and dipyridamole has a cilostazol:dipyridamole weight ratio of about 1:1 to about 1:12, or about 1:1.25 to about 1:12. Levels in blood can be measured by high performance liquid chromatography or by other methods known in the art.

In addition, the dosages of cilostazol and adenosine uptake inhibitors to be administered in the use of the present invention will vary depending upon, for example, the particular symptoms and illnesses to be treated, the mode of administration, and the age, weight and sex of the patient to be treated. Indeed, because individual patients may present a wide variation in severity of symptoms and illnesses, and each drug has its unique therapeutic characteristics, the precise mode of administration and dosages employed for each patient is left to the discretion of the practitioner.

"Patient" as used herein refers to any person or non-human animal in need of treatment for the above symptoms and illnesses, or to any subject for whom treatment may be beneficial, including humans and non-human animals. Such non-human animals to be treated include all domesticated and feral vertebrates, preferably, but not limited to: mice, rats, rabbits, fish, birds, hamsters, dogs, cats, swine, sheep, horses, cattle and non-human primates.

Pharmaceutical compositions according to the present invention comprise formulations of active ingredients (that is, the combination of cilostazol or pharmaceutically acceptable salt thereof and at least one adenosine uptake inhibitor or pharmaceutically acceptable salt thereof) together with one or more pharmaceutically acceptable carriers or excipients and optionally other therapeutic agents. The carrier(s) must be acceptable in the sense of being compatible with the other ingredients of the composition and not deleterious to the recipient thereof. When the individual components of the combination are administered together or separately they are generally presented as a pharmaceutical formulation.

Suitable formulations include those suitable for oral, rectal, nasal, topical (including transdermal, buccal and sublingual), vaginal or parenteral (including subcutaneous, intramuscular, intravenous and intradermal) administration. The formulations may be prepared by any methods well known in the art of pharmacy, for example, using methods such as those described in Gennaro et al., Remington's Pharmaceutical Sciences (18th ed., Mack Publishing Company, 1990, see especially Part 8: Pharmaceutical Preparations and their Manufacture). Such methods include the step of bringing into association the active ingredients with the carrier which constitutes one or more accessory ingredients. Such accessory ingredients include those conventional in the art, such as, fillers, binders, diluents, disintegrants, lubricants, colorants, flavoring agents, and wetting agents.

Formulations suitable for oral administration may be presented as discrete units such as pills, tablets or capsules each containing a predetermined amount of active ingredients as a powder or granules or as a solution or suspension. The active ingredients may also be present as a bolus or paste, or may be contained within liposomes.

Formulations for rectal administration may be presented as a suppository or enema.

For parenteral administration, suitable formulations include aqueous and nonaqueous sterile injection. The formulations may be presented in unit-dose or multidose containers, for example, sealed vials and ampules, and may be stored in a freeze dried (lyophilized) condition requiring only the addition of the sterile liquid carrier, for example, water prior to use.

Formulations suitable for administration by nasal inhalation include fine dusts or mists which may be generated by means of metered dose pressurized aerosols, nebulizers or insufflators.

The present invention further includes a process for the preparation of a pharmaceutical composition which comprises bringing into association a combination of cilostazol (or pharmaceutically acceptable salt thereof) and at least one adenosine uptake inhibitor (or pharmaceutically acceptable salt thereof) with one or more pharmaceutically acceptable carriers therefor.

The present invention is illustrated by the following Examples, which are not intended to be limiting in any way.

## Example 1

### Cilostazol and Dipyridamole Synergistically Inhibit the Aggregation of Human Washed Platelets *In Vitro*

#### Preparation of washed platelets

Peripheral blood samples were collected from ten healthy volunteers (medication-free for at least 10 days) by a two-syringe technique using a 19G butterfly needle. The procedure for drawing blood was approved by institutional review committee according to the Helsinki convention. Nine volumes of blood were directly collected into a syringe containing 1 volume of trisodium citrate (3.8%). Platelet rich plasma (PRP) was collected following centrifugation at 150 x g for 15 minutes at room temperature. Washed platelet (WP) suspension was prepared from citrated PRP by the citrate wash method as described previously in Cone et al. (Cone et al., 1999a). Platelets were finally re-suspended in Tyrode's HEPES buffer (136.7 mM NaCl, 5.5 mM dextrose, 2.6 mM KCl, 13.8 mM $NaHCO_3$, 1 mM $MgCl_2$, 0.36 mM $NaH_2PO_4$, and 10 mM HEPES; pH 7.4). Platelet concentration was adjusted to 3.8 x $10^8$ platelets/ml.

#### Description of test compounds

Cilostazol (OPC-13013): A MPDEI that selectively inhibits PDE3 and prevents platelet aggregation by elevating cAMP levels. (Provided by Otsuka Pharmaceutical Co. Ltd., Tokushima, Japan, Lot# B8E88M.)

Dipyridamole: An antiplatelet drug that blocks the uptake of adenosine into vascular and blood cells. (Calbiochem, La Jolla, CA., Cat# 322328, Lot# B11755.) ZM241385: A selective adenosine $A_{2A}$ receptor blocker. (Tocris, Ballwin, MO., Cat# 1036, Batch# 2/18074.)

#### Detection of washed platelet aggregation

Aggregation was quantified by the change in light transmission using an AG-10 Aggregation Analyzer (Kowa, Japan). Washed platelets were maintained at room temperature and the study was performed within 3 hours following blood collection. Cilostazol was dissolved in DMSO and adenosine was dissolved in water. Appropriate dilutions were made to obtain desired working concentrations while maintaining the final concentration of DMSO at no more than 0.2%. The platelet suspension (400 $\mu$l) was pipetted into an aggregation cuvette and allowed to incubate with stirring at 1,000 rpm at 37°C for 1 minute. Drug or vehicle (DMSO) was then added (0.4 $\mu$l) and incubated for another 3 minutes. When testing for synergism with dipyridamole, dipyridamole (1, 3, and 10 $\mu$M) and 1 $\mu$M adenosine were added 1 minute following the addition of drug or DMSO so that the overall incubation time for dipyridamole and adenosine was 2 minutes. Then, the suspension was stimulated with 1-2 $\mu$g/ml collagen (Chrono-Log Corp., Havertown, PA). The overall time of aggregation recorded was 15 minutes. Maximal light transmission values during the last 11 minutes (after the addition of collagen) are presented as the percentage of control aggregation (DMSO + ethanol + adenosine).

Cilostazol and dipyridamole synergistically inhibit the aggregation of washed platelets

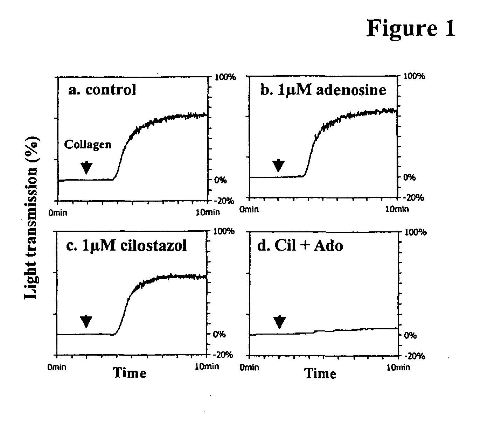

To study the synergistic effect of adenosine and cilostazol on the aggregation of washed platelets, the amount of collagen was titrated for each individual donor in the presence of 1 $\mu$M adenosine. The minimum concentration of collagen (1-5 $\mu$g/ml) in which 1 $\mu$M adenosine showed no effect on aggregation was used. Cilostazol (1 $\mu$M) or adenosine (1 $\mu$M) by itself had little effect on collagen-induced platelet aggregation (Figure 1a, 1b, 1c). However, combining both completely inhibited platelet aggregation (Figure 1d).

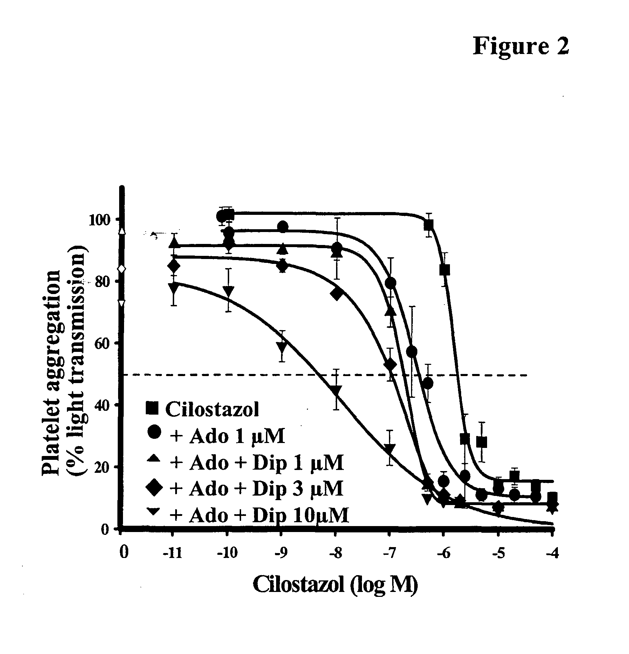

The concentration-dependent inhibition of collagen-induced aggregation of washed platelets by cilostazol in combination with dipyridamole (1, 3 and 10 $\mu$M) was performed in the presence of adenosine (1 $\mu$M). As shown in Figure 2, cilostazol dose-dependently inhibited platelet aggregation. Addition of adenosine shifted the inhibitory curve to the left (Table 2). The calculated $IC_{50}$ was reduced from 2.66 $\pm$ 0.41 $\mu$M to 0.38 $\pm$ 0.05 $\mu$M ($p < 0.001$, two-tails paired Student $t$-test). Dipyridamole dose-dependently shifted the inhibitory curves of cilostazol with adenosine further to the left. The $IC_{50}$ was shifted to 0.17 $\pm$ 0.04 $\mu$M ($p < 0.05$), 0.11 $\pm$ 0.66 $\mu$M ($p < 0.05$), and 0.01 $\pm$ 0.01 $\mu$M ($p < 0.005$) in the presence of 1, 3, and 10 $\mu$M dipyridamole, respectively (Table 1). The data indicate that combination of dipyridamole and cilostazol exerted a synergistic effect on the inhibition of platelet aggregation, rather than an additive effect.

Table 1

| $IC_{50}$ of Cilostazol on Platelet Aggregation | | | | |
|---|---|---|---|---|
| **Cilostazol** | **+Adenosine (1 $\mu$M)** | **+Adenosine +Dipyridamole (1 $\mu$M)** | **+Adenosine +Dipyridamole (3 $\mu$M)** | **+Adenosine +Dipyridamole (10 $\mu$M)** |
| 2.66$\pm$0.41$\mu$M (n=6) | 0.38$\pm$0.05$\mu$M (n=5) | 0.17$\pm$0.04$\mu$M (n=5) | 0.11$\pm$0.06$\mu$M (n=5) | 0.01$\pm$0.01$\mu$M (n=5) |
| | p<0.001 (vs. w/o Ado) | p<0.05 (vs. w/ Ado) | p<0.05 (vs. w/Ado) | p<0.005 (vs. w/Ado) |

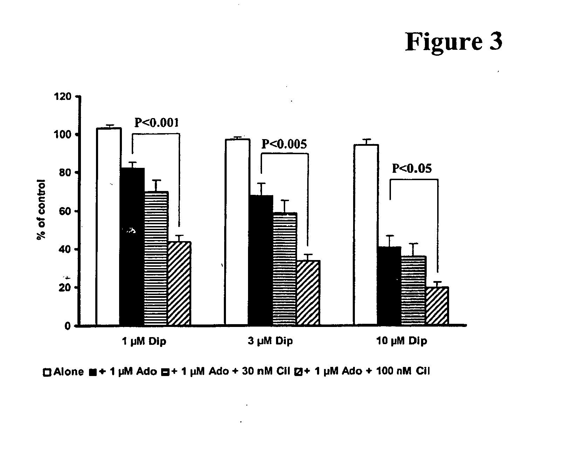

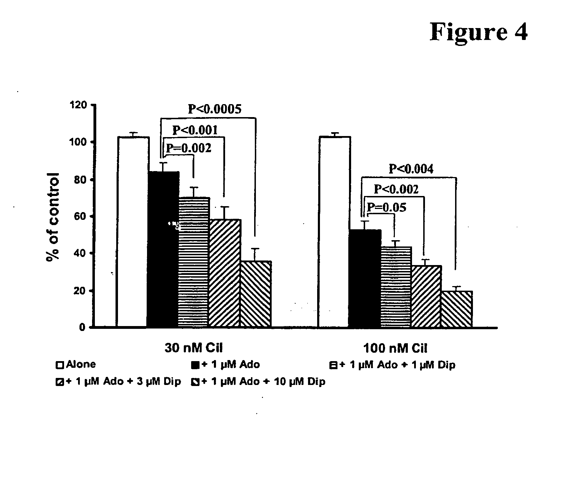

The synergistic effect of cilostazol and dipyridamole was reconfirmed using washed platelets from five additional donors but this time, with focus on 30 and 100 nM cilostazol. Figure 3 shows the % inhibition of platelet aggregation compared with control (no drugs added) when 1 and 3 $\mu$M cilostazol was added in the presence of adenosine (1 $\mu$M) and dipyridamole (1, 3 and 10 $\mu$M). Compared with controls, dipyridamole (1, 3 or 10 $\mu$M) or cilostazol (30 or 100 nM) alone had no significant inhibitory effect on washed platelet aggregation at the concentrations tested (Figure 3 and 4). Addition of adenosine (1 $\mu$M, at which no effect by adenosine alone was observed) enhanced the effect of dipyridamole significantly (Figure 3). Further enhancement was observed with the addition of 100 nM but not 30 nM cilostazol. Therefore, the inhibitory effect of 10 $\mu$M dipyridamole on platelet aggregation could be achieved with the combination of 1 $\mu$M dipyridamole and 100 nM cilostazol, in the presence of adenosine, due to synergistic effect between the two compounds. Figure 4 shows the % inhibition of platelet aggregation compared to control when 1, 3 or 10 $\mu$M dipyridamole was added in the presence of 1 $\mu$M adenosine and 30 or 100 nM cilostazol. The combination of 30 or 100 nM cilostazol with 1 $\mu$M adenosine showed significant differences from controls but not either alone. Addition of dipyridamole to the combination at both cilostazol concentrations (30 and 100 nM) significantly enhanced the inhibitory effect at all three concentrations. Again, the synergistic effect between the two compounds can be illustrated in that the equivalent effect of 100 nM cilostazol could be achieved by the combination of 30 nM cilostazol with 3 $\mu$M dipyridamole, in the presence of adenosine. As expected, the combination of cilostazol with dipyridamole without adenosine had no effect on washed platelet aggregation (data not shown), suggesting that adenosine is the mediator of the synergistic effect between cilostazol and dipyridamole. Therefore, these experiments clearly demonstrated that the combination of cilostazol and dipyridamole synergistically inhibits platelet aggregation. This would allow the use of much lower concentrations of each agent in combination to achieve the same efficacy as that obtained with higher concentrations of each agent used alone. The synergistic effect was believed to be due to enhanced elevation of intracellular cAMP levels, as demonstrated below.

## Example 2

### Cilostazol and Dipyridamole Synergistically Increase the Concentration of Intracellular cAMP

#### Measurement of cAMP in platelets

Adenosine, cilostazol, or dipyridamole alone or in combination was first aliquoted into separate polypropylene test tubes. DMSO and ethanol were used as controls. Test agents alone or in combinations were mixed with PRP by brief vortexing. The final sample volume was 200 $\mu$l and each experiment was performed in duplicates. After incubating the samples at 37°C for 5 minutes, the reaction was terminated by adding 50 $\mu$l of ice-cold perchloric acid (PCA, 1.25N). After freezing and thawing once, the mixture was neutralized with 50 $\mu$l of $KHCO_3$ (1.25N) and centrifuged at 20,000 x g for 15 min at 4°C. The resulting supernatants were collected and diluted with acetate buffer provided with the kit. The cAMP concentration was measured in duplicates using a cAMP radioimmunoassay kit (NEK-033, NEN Life Science, Boston, MA).

#### Establishment of CHO cells expressing human adenosine $A_{2A}$ receptor

Total RNA was extracted from fresh human platelets and 5 $\mu$g were reverse-transcribed into cDNA and used as a template for the polymerase chain reaction (PCR). Specific primers with a Kozak sequence (CCCACC) for adenosine $A_{2A}$ receptor were designed (forward primer: 5'-CCCACCATGCCCATCATGGGCT-3', reverse primer: 5'-TCAGGACACTCCTGCTCC-3') and synthesized by Life Technologies (Rockville, MD). Using these primers, full coding regions were amplified by PCR and further recombined into the cloning vector, pCR2.1 (Invitrogen, Carlsbad, CA). The DNA sequence of the insert was confirmed before inserted into the mammalian expression vector, pcDNA3.1+ (Invitrogen). An expression vector (pCRE-Luc) containing a cAMP-response element (CRE) in the promoter region, which drives the expression of luciferase, was purchased from Stratagene (La Jolla, CA). The level of luciferase expression reflects the concentration of intracellular cAMP. It is known that adenosine $A_{2A}$ receptor is coupled to $G_s$ proteins (Huttemann et al., 1984). Therefore, the activation of the receptors would be reflected by luciferase expression, where the expression level can be measured by the luciferase activity assay. Co-transfection of the luciferase reporter vector with the vectors containing adenosine $A_{2A}$ receptors was carried out by calcium phosphate precipitation into Chinese hamster ovary (CHO) cells. Stable transfectants were selected with 1.0 mg/ml G418 (Life Technologies) for 12 days. The cell clones over-expressing functional adenosine $A_{2A}$ receptors were determined by luciferase expression under the stimulation of adenosine.

#### Luciferase assay

To test the synergistic effect of cilostazol and dipyridamole with or without adenosine on cAMP elevation, the cells were sub-cultured at near-confluence into a white-wall 96-well plate with clear bottom (Coming Costar Co., Cambridge, MA). The next day, the cells were washed once with F12K medium supplemented with 0.5% FCS and then incubated with 100 $\mu$l of the medium only (basal) or medium plus test agents for 4 hours at 37°C. After equilibrating to room temperature, 100$\mu$l of detection substrate (Bright-Glo™ luciferase assay system, Promega, Madison, WI) were added to each well. The luciferase activity was measured after 5 minutes using a Mediators PhL luminescence plate reader (ImmTech, New Windsor, MD). The value of luminescence (arbitrary unit) detected during half a second was taken as luciferase activity.

#### Cilostazol and dipyridamole synergistically enhance the intracellular levels of cAMP

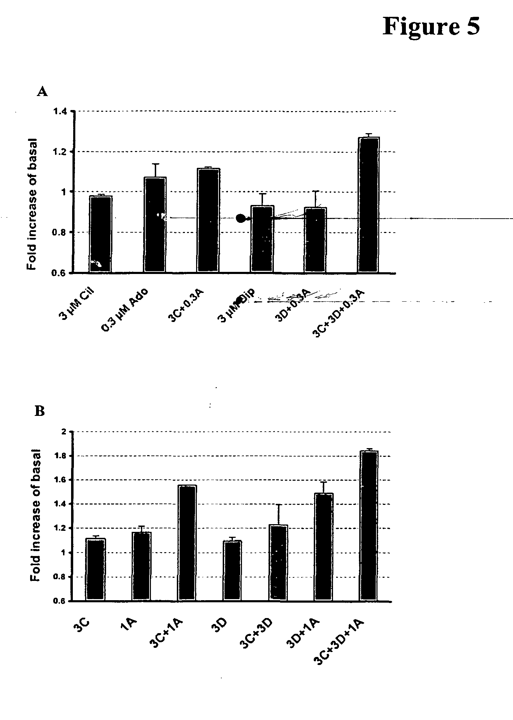

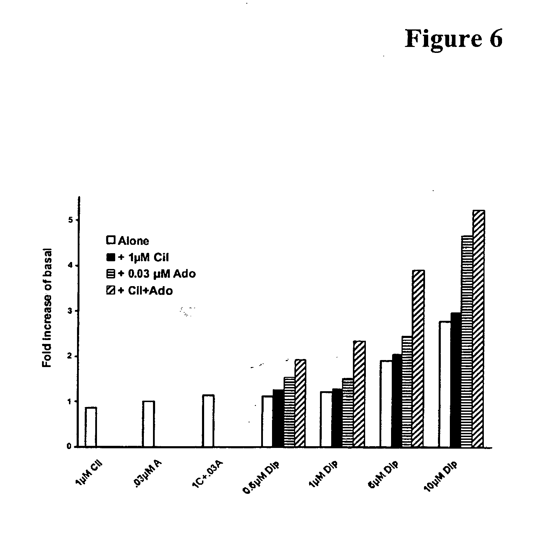

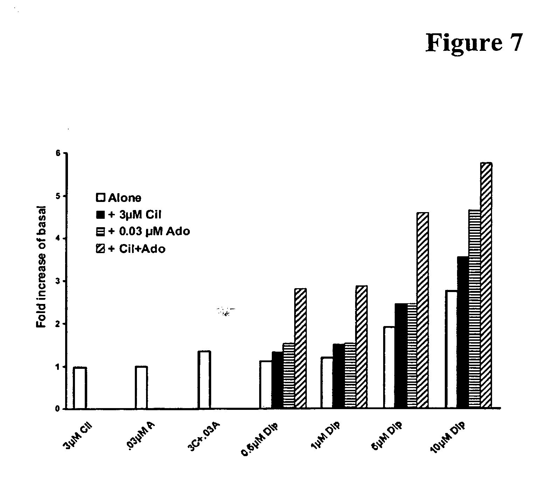

The effect of dipyridamole and cilostazol on intracellular cAMP concentration was first studied in PRP. As shown in Figure 5A and 5B, in the presence of 0.3 or 1 $\mu$M adenosine, dipyridamole (3 $\mu$M) in combination with cilostazol (3 $\mu$M) further increased intra-platelet cAMP levels, when compared with either alone (n = 2 of duplicate assays). Because of the low basal cAMP levels in platelets, we established a luciferase assay in CHO cells which over-expressed the human platelet adenosine $A_{2A}$ receptor. The amount of luciferase activity reflects intracellular cAMP levels. The inhibitory effect of dipyridamole on adenosine uptake was similar in platelets and erythrocytes. Figure 6 shows the effect of dipyridamole on luciferase activity in the presence of 0.03 $\mu$M adenosine and/or 1 $\mu$M cilostazol. Similarly, Figure 7 shows the effect of dipyridamole on luciferase activity in the presence of 0.03 $\mu$M adenosine and/or 3$\mu$M cilostazol (representatives in triplicates of at least 3 independent experiments). As shown in Figure 6 and 7, 1 and 3 $\mu$M cilostazol

synergistically elevated luciferase activity in the presence of dipyridamole, even in the absence of adenosine in the case of 3 $\mu$M cilostazol. Dipyridamole dose-dependently enhanced the effect of cilostazol in the range of 0.5 $\mu$M to 10 $\mu$M, peaking at about 5 $\mu$M. Overall, these studies establish that cilostazol and dipyridamole act synergistically to enhance the intracellular concentration of cAMP, and they provide a likely mechanism by which these agents synergistically inhibit platelet aggregation.

## Example 3

### Cilostazol Inhibits the Uptake of Adenosine

Assay for adenosine uptake into washed platelets and erythrocytes

Washed erythrocytes (wRBC) were prepared as follows. After initial centrifugation and removal of PRP and buffy coat, 100 $\mu$l of the red pellet portion were diluted into 12 ml PBS containing calcium and magnesium. RBC were spun at 150 x g for 5 min. After one more wash with PBS, the pellet was resuspended in PBS to 1 x $10^8$ RBC/ml. Adenosine uptake experiments were performed according to the method described previously (Liu, Fong, Cone, Wang, Yoshitake, and Kambayashi, 2000). 100 $\mu$l WP or wRBC were incubated with 50 $\mu$l of cilostazol or milrinone at 37°C for 5 min. Then, 50 $\mu$l of 1 $\mu$Ci of [$^3$H]-adenosine (Amersham Pharmacia, Piscataway, NJ), 1 $\mu$M adenosine, and 25 $\mu$M erythro-9-(2-hydroxy-3-nonyl)adenosine (EHNA, final concentration, Sigma Chemical) was added, followed by 200 $\mu$l oil (dibutyl phthalate : dioctyl phthalate = 1 : 1, Aldrich) and then incubated for 1 min. The cells were separated from free adenosine in the water phase by centrifugation at 16,000 x g for 2 min. After removing the oil and water phases, the radioactivity of the cell pellet was measured using a $\beta$-liquid scintillation counter (1209 Rackbeta, LKB, Turku, Finland).

Cilostazol inhibits adenosine uptake

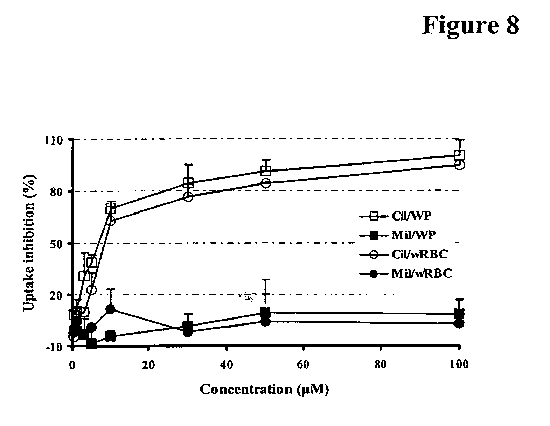

[$^3$H]adenosine uptake experiments were performed with washed platelets and washed erythrocytes and the results are shown in Figure 8. Cilostazol inhibited adenosine uptake in both platelets and erythrocytes with an IC$_{50}$ of about 7 $\mu$M (n = 3). The potency of cilostazol on the uptake inhibition is similar to the values reported previously on rabbit cardiac myocytes, human vascular smooth muscle, and endothelial cells (5~10 $\mu$M) (Liu, Fong, Cone, Wang, Yoshitake, and Kambayashi, 2000). In contrast, milrinone had virtually no effect on adenosine uptake by platelets or erythrocytes. CHO cells over-expressing functional human A$_{2A}$ receptors were used to further confirm the role of cilostazol in inhibiting the adenosine uptake. Cilostazol inhibited [$^3$H]adenosine uptake into these CHO cells with similar potency to platelets and erythrocytes, while milrinone had no effect (data not shown).

## Example 4

### Cilostazol and Dipyridamole Synergistically Inhibit the Aggregation of Platelets in Human Whole Blood *In Vitro*

Preparation of whole blood

Peripheral blood samples were collected from ten healthy volunteers (medication-free for at least 10 days) by a two-syringe technique using a syringe containing 4 $\mu$l of hirudin (250 U/$\mu$l)/10ml of blood.

Whole blood platelet aggregation study

Blood samples were diluted 1:1 with physiological saline and tests were performed using a Chrono-Log Whole Blood Aggregometer with a stirring rate of 1000 rpm. At the start, a stirring bar was dropped into a plastic cuvette followed by the addition of 1 ml diluted whole blood. The electrodes were then placed in the cuvette and the sample was allowed to incubate at 37°C while the instrument was calibrated. Cilostazol and ZM241385 were dissolved in DMSO to a stock concentration of 100 mM. Dipyridamole was diluted in ethanol (EtOH) to a stock concentration of 100 mM. Further dilutions were made so that the appropriate testing concentration of cilostazol (10 $\mu$M and 30 $\mu$M, because of the binding property of cilostazol to protein), dipyridamole (0.1, 0.3, 1 or 3 $\mu$M), and ZM241385 (0.1 $\mu$M) would be obtained when added to the 1 ml of whole blood. Drug and vehicle were added in a volume of 1 $\mu$l so that the final concentration of DMSO did not exceed 0.2%. The suspension was allowed to incubate for 3 minutes before collagen was added. The collagen concentration used in this study was 0.5 $\mu$g/ml, determined by preliminary screening. To test the synergism between cilostazol and dipyridamole, dipyridamole (1 $\mu$l stock) was added 1 minute after the addition of cilostazol. To see the reverse effects of these drugs, 0.1 $\mu$M of ZM241385 (1 $\mu$l) was added 1 minute before the addition of the drugs. Collagen was added 3 minutes after the addition of drugs, so the ZM241385 was allowed to incubate for a total of 4 minutes, cilostazol or DMSO 3 minutes, and dipyridamole 2 minutes. After stimulation, the amplitude was observed for 11 minutes with maximal amplitude used for data presentation. To test whether lower concentrations of cilostazol can also synergize with dipyridamole to inhibit platelet aggregation in whole-blood, we stimulated platelets with slightly lower concentrations of collagen (0.1 or 0.3 $\mu$g/ml) that produce less potent aggregation but are more relevant to conditions in patients. Different combinations of cilostazol (0.3, 0.7, 1 and 3 $\mu$M) and dipyridamole (1 and 3 $\mu$M) were examined. Data are expressed as percent of the values detected in the absence of any inhibition.

Measurement of adenosine concentration in plasma

Blood was drawn and mixed with recombinant human huridin (100 U/ml). The same procedure for platelet aggregation was used to stimulate these platelets with collagen (2 $\mu$g/ml). After a 5-minute incubation, 500 $\mu$l of WB were mixed quickly with 500 $\mu$l of ice-cold saline. The cells were spun at 20,000 x g for 4 minutes at 4°C. Supernatant (600 $\mu$l) was first mixed with 300 $\mu$l PCA (2.5 N) and then neutralized with 300 $\mu$l of KHCO$_3$ (2.5 M). Finally, the mixture was centrifuged at 20,000 x g for 15 minutes at 4°C. The adenosine concentration in the supernatants was measured using reverse-phase high performance liquid chromatography (HPLC, Waters Alliance 2690) with a Hypersil 3$\mu$ C$_{18}$ column (150mm x 4.6mm) and a gradient from 5 to 20 % methanol in 20 mM KH$_2$PO$_4$. Adenosine was detected using a diode array detector (Water 996) with an absorbance change at 258 nm and quantified by comparison of retention times and peak height with those of a known external standard. Quantification was performed using Waters Millennium 32 Client/Server software.

Large amounts of adenosine are generated in whole blood during platelet activation

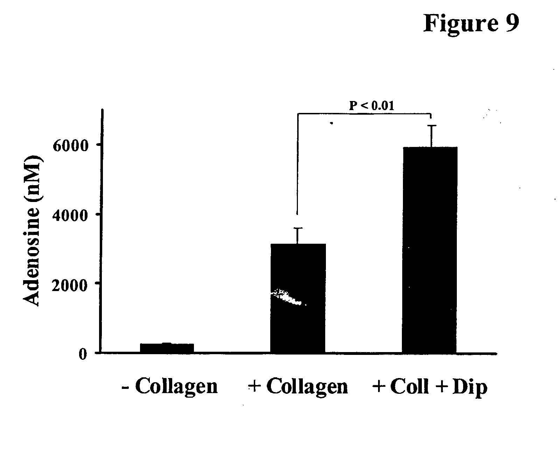

Using HPLC, adenosine concentrations in the extracellular medium of whole blood were measured 5 minutes after stimulating with 2 $\mu$g/ml collagen. As shown in Figure 9, a large amount of adenosine (3152 $\pm$ 428 nM, compared to basal 240 $\pm$ 53 nM, n = 5) was generated in whole blood after collagen stimulation, probably due to the degradation of released ATP and ADP from activated platelets. In the presence of dipyridamole (1 $\mu$M), platelet aggregation was not affected, but adenosine levels increased significantly further to 5916 $\pm$ 641 nM (n = 3).

Cilostazol and dipyridamole synergistically inhibit platelet aggregation in whole blood

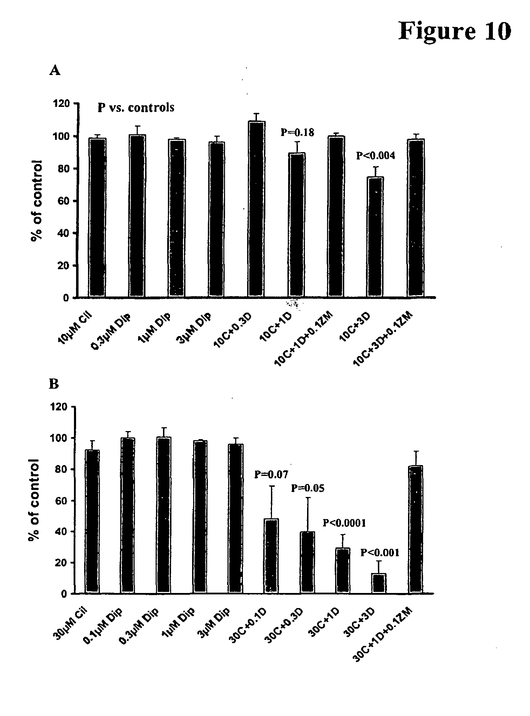

As observed above, it is not necessary to add any exogenous adenosine to this assay because large amounts of adenosine can be generated during platelet activation. In whole blood, experiments have shown that cilostazol (10 or 30 $\mu$M) or dipyridamole (0.1, 0.3, 1 or 3 $\mu$M) alone did not have a significant effect on platelet aggregation (Figure 10). However, the combination of 10 $\mu$M cilostazol and 3 $\mu$M dipyridamole significantly inhibited platelet aggregation (from 98.9 $\pm$ 2.0% for 10 $\mu$M cilostazol alone and 97.9 $\pm$ 0.7% for 3 $\mu$M dipyridamole alone to 74.8 $\pm$ 6.2%, n = 8, p < 0.005, Figure 10A). Clearer demonstration was seen with the combination of 30 $\mu$M cilostazol with dipyridamole at even lower concentrations (n = 5 to 14, Figure 10B). The synergistic effect was dose-dependent for both cilostazol and dipyridamole. Additionally, in the presence of the ZM241385 (0.1 $\mu$M), a selective adenosine A$_{2A}$ receptor antagonist, the synergistic effect of dipyridamole and cilostazol reverted back to the basal level of cilostazol alone (n = 8), suggesting that the synergistic effect was mediated by the accumulation of adenosine in the plasma.

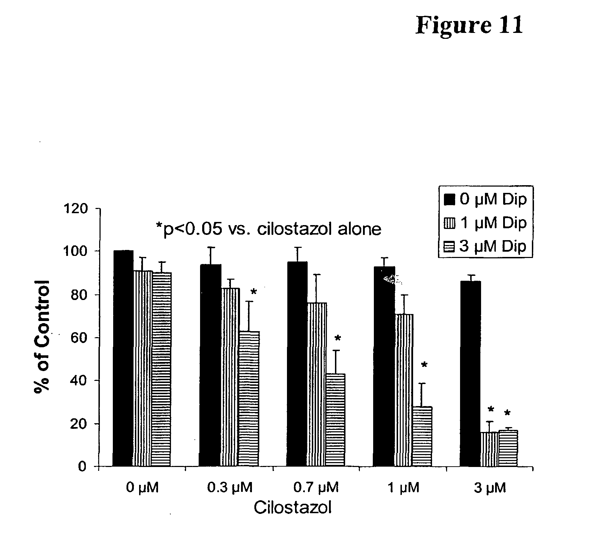

When whole-blood aggregation was induced by 0.1 or 0.3 $\mu$g/ml of collagen, we observed that combination of cilostazol (between 0.3 $\mu$M to 3 $\mu$M) and dipyridamole (1 or 3 $\mu$M) significantly inhibited platelet aggregation (Figure 11). For example, a combination of 0.7 $\mu$M cilostazol and 3 $\mu$M dipyridamole inhibited platelet aggregation by 57$\pm$11%, and a combination of 1 $\mu$M cilostazol and 3 $\mu$M dipyridamole inhibited platelet aggregation by 72$\pm$11% (p<0.001). Cilostazol or dipyridamole alone at these concentrations did not cause any significant inhibition.

## **Example 5**

**Cilostazol and Dipyridamole Synergistically Inhibit the Aggregation of Platelets in Human Whole Blood *Ex Vivo***

Design of clinical study

A one-period, open label, sequential, crossover study was designed to test whether a synergistic effect of cilostazol and dipyridamole on inhibition of platelet aggregation can be observed at clinically relevant doses in healthly volunteers. Six subjects received one 100 mg tablet of cilostazol (Pletal®) on study day one. On study day 4, subjects received one 200 mg tablet of dipyridamole. On study day 6, these subjects received the cilostazol and dipyridamole combination.

Whole blood platelet aggregation

Prior to, and 2 and 4 hours after dosing, 5 ml of blood were drawn into a syringe containing 10 U/ml of fractionated heparin. Blood samples were then diluted with physiological saline and platelet aggregation was measured using a Chrono-Log Whole Blood Aggregometer with a stirring rate of 1000 rpm. The platelet aggregation was induced by the addition of collagen (final concentration of 0.3 $\mu$g/ml, Nycomed Arzneimittel, Munchen, Germany). The percentage aggregation was recorded at each time point. To compare the effect of drug treatments, the aggregation at 2-hour and 4-hour is normalized as a percentage to the values prior to dosing.

The combination of cilostazol and dipyridamole synergistically inhibit *ex vivo* whole blood platelet aggregation

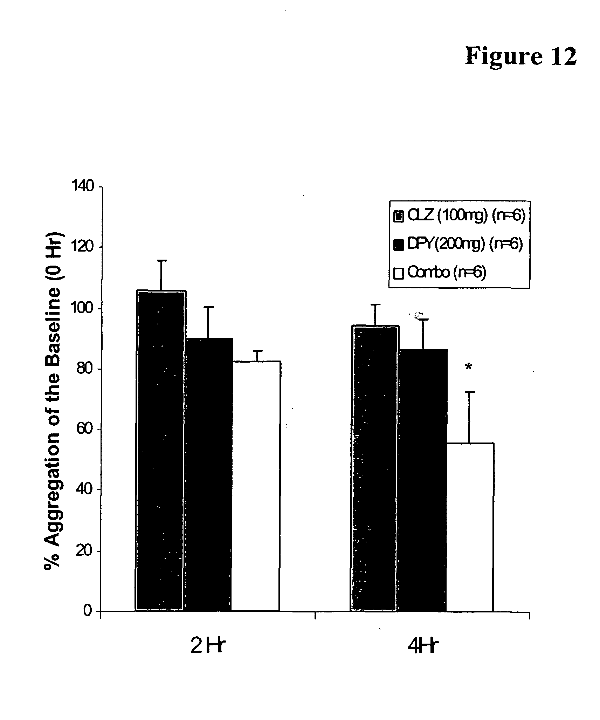

The blood concentration of cilostazol at 2- and 4-hours after a single dose of 100 mg is about 2 $\mu$M. Based on previous pharmacokinetic data, the blood concentration of dipyridamole at 2- and 4-hours after a single dose of 200 mg is also in the range of 2 $\mu$M. Because there is a requirement for 1:1 dilution of blood with saline according to the Aggregometer manufacturer's instructions, the effective drug concentrations in the *ex vivo* platelet aggregation assay are estimated to be 1 $\mu$M for cilostazol and 1 $\mu$M for dipyridamole. As expected, at these concentrations, neither cilostazol nor dipyridamole alone inhibited platelet aggregation (Figure 12). However, platelet aggregation is inhibited by 45% at 4-hours after subjects were treated with the combination of cilostazol and dipyridamole ($p < 0.001$ vs. prior dosing). These results are very similar to the data obtained in the *in vitro* whole blood aggregation studies described in Example 4.

## Example 6

### Dipyridamole Counteracts the Potentially Deleterious Effects of Cilostazol on Cardiac Function

Surgical preparations

This study was conducted in accordance with the "Guide for the Care And Use of Laboratory Animals", published by the National Research Council, 1996, Washington DC, and approved by the Institutional Animal Care and Use Committee of Otsuka Maryland Research Institute, LLC. Male rabbits (New Zealand White), weighing 2 - 2.5 kg, were anaesthetized with intravenous pentobarbital (30 mg/kg) through a marginal ear vein. A tracheotomy was performed and the animals were intubated. Ventilation was with room air supplemented with 100% $O_2$ via a Harvard small animal ventilator. The respiratory rate was adjusted to keep arterial blood $PO_2$, $PCO_2$ and pH in the physiological range. Body temperature was maintained near 38°C with a heating blanket. Hearts were exposed through a mid-line incision of the chest, and quickly excised by an incision at the base of the heart and put into ice-cold Krebs-Henseleit bicarbonate buffer. The heart was then attached to a Langendorff apparatus by the aortic root, and perfused with non-recirculating Krebs-Henseleit buffer at a constant pressure of 75 mmHg. The perfusate was bubbled with 95% $O_2$ and 5% $CO_2$ gas mixture, and the bubbling rate was adjusted to maintain physiological pH (7.35-7.45). Perfusate temperature was maintained at 38°C by a circulating water-jacket surrounding the buffer reservoirs. The heart was also maintained at 38°C via a water-jacketed housing in which it was suspended. The open top of the jacket was covered with a piece of parafilm to maintain the humidity and temperature. The pulmonary artery around the right side of the aortic root was cannulated for collecting coronary effluent and for coronary flow rate measurement with a graduated cylinder. A saline-filled latex balloon, connected via a catheter to a pressure transducer, was inserted into the left ventricle and inflated to yield an end-diastolic pressure of 0 - 5 mmHg. The pressure transducer was connected to a Grass Chart Recorder (Model 7) to record left ventricular pressure and its first derivative (dp/dt), and heart rate. Hearts with left ventricular developing pressure less than 85 mmHg at the end of the 15-min equilibrium period were not included in the study.

Cardiac function measurements

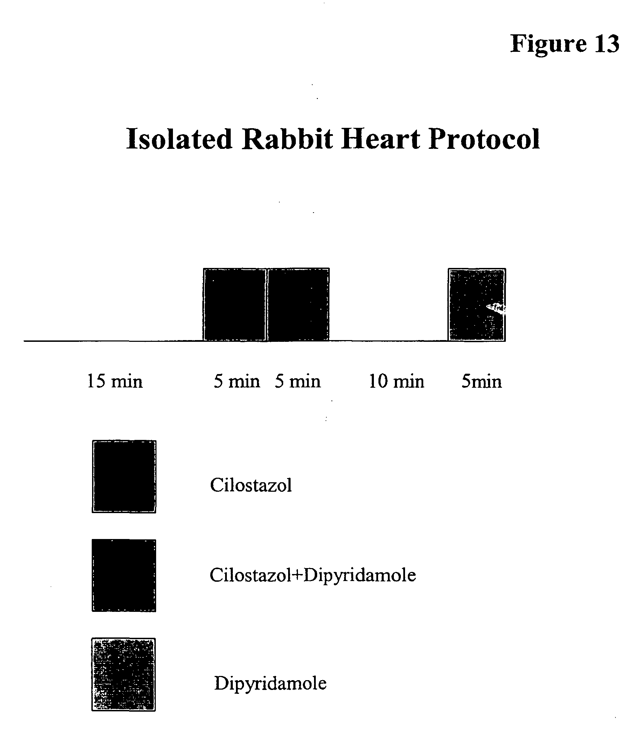

The cardiac function indexes measured were LVDP (left ventricular developed pressure), dp/dt$_{max}$ (the maximal value of the first derivative of the LVDP), heart rate, and coronary flow. The experimental protocol is shown in Figure 13. After a 15-min equilibrium, hearts were treated with cilostazol for 5 min, followed by 5 min of cilostazol and dipyridamole. After 10 min of drug-free perfusion, hearts were treated for 5 min with dipyridamole. Measurements for cardiac function were taken at the end of each 5 min drug treatment. The effect of drug treatment is expressed as the percent change of values before and after each drug treatment:

$$\text{\% Change from Baseline}$$

$$= [(\text{Value after drug-Value before drug})/\text{Value before drug}] \times 100$$

Statistical analysis

Data are presented as mean $\pm$ SEM. A paired *t*-test was used to detect the significance (p < 0.05) (Sigma Stat 2.0, Jandel Corporation, San Rafael, CA)

Dipyridamole counteracts cilostazol-induced increases in cardiac contractility and heart rate

Previous studies revealed that cilostazol has minimal effects on cardiac function at concentrations below 1 $\mu$M. It has also been shown that dipyridamole is a very potent and effective adenosine uptake inhibitor at concentrations of 0.3 to 1 $\mu$M. Therefore, these experiments were performed using cilostazol concentrations of 1, 3 and 10 $\mu$M, and dipyridamole concentrations of 0.3, 1, and 3 $\mu$M.

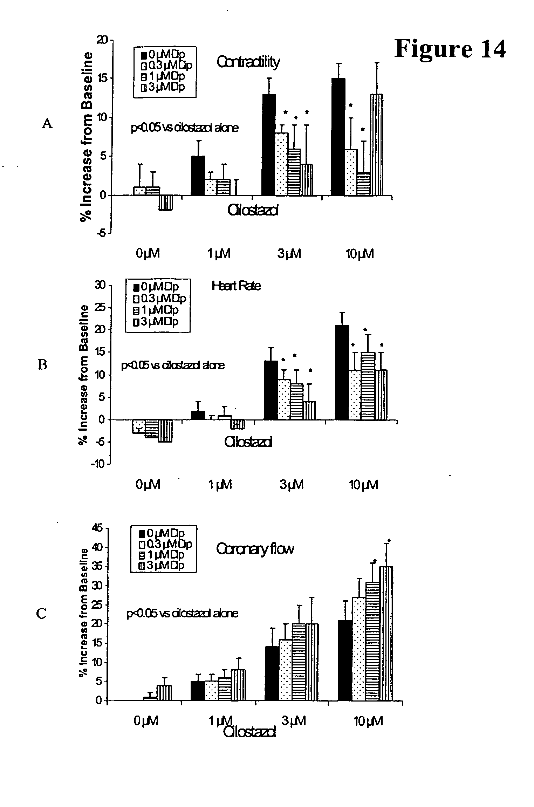

As expected, dipyridamole at 0.3, 1 or 3 $\mu$M alone had no significant effect on cardiac function. However, cilostazol at 3 or 10 $\mu$M significantly increased cardiac contractility, heart rate and coronary flow. Dipyridamole at 0.3, 1 or 3 $\mu$M significantly reduced the cilostazol-induced increase of cardiac contractility (Figure 14A) and heart rate (Figure 14B). Dipyridamole at 1 and 3 $\mu$M also augmented the cilostazol (10 $\mu$M)-induced increase in coronary flow (Figure 14C). In conclusion, this study suggests that dipyridamole may counteract the potential deleterious effects of cilostazol on cardiac function.

## Example 7

**Combination of Low Levels of Cilostazol and Dipyridamole Increases Blood Flow in Gastrocnemius Muscle During Exercise and Improves Blood Flow Recovery After Ischemia**

This Example demonstrates that the administration of a combination of cilostazol and dipyridamole increases blood supply to exercised skeletal muscle and improves flow recovery after a period of ischemia *in vivo.* The hindlimbs of rabbits were prepared for drug infusion, stimulation of the limbs to mimic exercise, and blood flow measurement as described below.

Surgical preparations

Male rabbits (New Zealand White), weighing 2.5-3.5 kg, were anaesthetized with intravenous pentobarbital (30 mg/kg) through a marginal ear vein. A tracheotomy was performed and the animals were intubated. Ventilation was with room air supplemented with 100% $O_2$ via a Harvard small animal ventilator. Body temperature was maintained near 38°C with a heating blanket. The jugular vein was cannulated for additional anesthesia and drug administration. A Millar pressure transducer (Miller Instruments, Houston) with lumen (4F) was inserted into the left carotid artery and advanced to the left ventricle for left ventricular pressure (LVP) measurement and infusion of fluorescent microspheres. The right carotid artery was cannulated for arterial blood pressure measurement. The femoral arteries of both hindlimbs were exposed through a longitudinal skin incision in the medium thigh that extended from the inguinal ligament to the stifle. Arterial occlusion was realized with an artery clamp, and reperfusion was performed by removal of the clamp. To stimulate the muscle contraction, a pair of electrodes was placed on the sciatic nerve of the left hindlimb and the connected to a Grass SD9 stimulator. The stimulation was produced with an 8 ms square pulse of supramaximal 10 V at 1 Hz. The hindlimbs were positioned to a 90° degree with the thigh. The contralateral hindlimb served as a control and was not stimulated.

Regional blood flow determination

The blood flow was measured using fluorescent microspheres according to the "Manual for Using Fluorescent Microspheres to Measure Organ Perfusion" (Fluorescent Microsphere Resource Center, University of Washington, Seattle, WA). Fluorescent-labeled polystyrene microspheres (15 $\mu$m diameter) in blue-green, yellow-green, orange, red and crimson were purchased form Molecular probes (Eugene, OR). Half million per kg of body weight of each colored microsphere were injected into the left ventricle through the catheter in 20 seconds. Simultaneously, a blood sample was withdrawn from the right carotid artery at 2.5ml/min for 2 min, starting 30 seconds before the injection of microspheres. At the end, the rabbit was euthanized with a lethal dose of pentobarbital sodium (100mg/kg). Tissue samples (about 1g each piece) were taken from the left ventricular free wall, the kidney, and the gastrocnemius muscle of both hindlimbs. The samples were weighed, placed in tubes and processed for digestion and fluorimetry. The fluorescence was measured with a spectrofluorometer (Fluomax-2, Instruments S.A., Inc, Edison, New Jersey). The regional blood flow was calculated by the standard reference flow technique, and expressed as ml/min/100g.

Experimental protocols

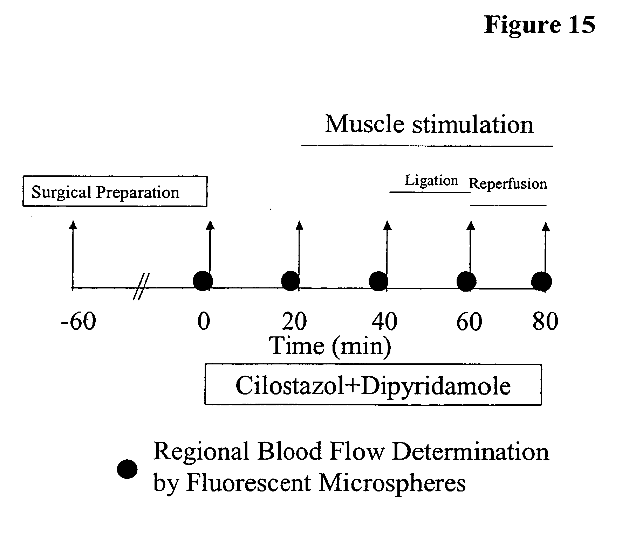

The time course of the experiment is shown in Figure 15. Sixty minutes after the surgical preparation, animals were divided into four groups and received either vehicle (control) or a combination of cilostazol (0.225mg/kg bolus followed by 0.0175mg/kg/min intravenously) and dipyridamole (20 $\mu$g/kg/min intravenously) (Cil+Dip). The drug infusion protocol was determined previously and produced blood concentrations of about 1 $\mu$M cilostazol and about 1 $\mu$M dipyridamole. Sixty minutes after the surgery, injection of vehicle or the drug combination was initiated. After 20 minutes, the gastrocnemius muscle of the left hindlimb was stimulated throughout the rest of the experiment. Twenty minutes after the stimulation (40 minute time point in Figure 15), the left femoral artery was clamped for 20 minutes to induce ischemia and then released to allow reperfusion. The regional blood flow was determined by the injection at 0, 20, 40, 60, and 80 minutes of blue-green, yellow-green, orange, red and crimson fluorescent microspheres.

Statistics

Data are presented as mean$\pm$SEM. P<0.05 was taken as the level of statistical significance (Sigma Stat 2.0, Jandel Corporation, San Rafael, CA). The data were analyzed by a two-way (group and time as variances) ANOVA (analysis of variance) with repeated measurements followed by a *post hoc* Student-Newman-Keuls test.

Combination of low levels of cilostazol and dipyridamole increases blood flow in exercised muscle and improves flow recovery after ischemia

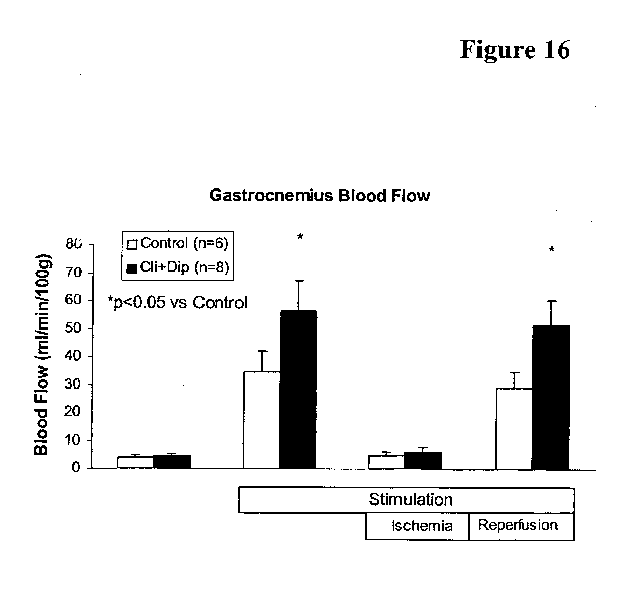

The administration of a combination of cilostazol and dipyridamole did not significantly alter the blood flow of resting gastrocnemius muscle. While stimulation significantly increased blood flow to the gastrocnemius muscle in both groups, the blood flow in the combination drug-treated muscle was significantly higher compared with that in the vehicle-treated muscle (from 35 $\pm$ 7 ml/min/100g in the vehicle-treated muscle to 56 $\pm$ 11 ml/min/100g in the combination drug-treated muscle, p<0.05) (Figure 16). The combination drug-treated muscle also had a significantly higher blood flow after a 20-minute complete ligation of left femoral artery (51 $\pm$ 9 ml/min/100g vs. 29 $\pm$ 6 ml/min/100g in the vehicle-treated muscle, p<0.05). The results suggest that the combination of cilostazol and dipyridamole increases blood supply to the exercise skeletal muscle and improves flow recovery after a period of ischemia.

The above Examples demonstrate that an adenosine uptake inhibitor can reduce the positive inotropic and chronotropic effects of a PDE3 inhibitor. Moreover, the Examples demonstrate that the combination of the MPDEI with an adenosine uptake inhibitor results in synergistic reduction of platelet aggregation, and thus can be used at lower concentrations than with either agent alone, without adversely affecting cardiac contractility. The Examples also demonstrate that a combination of low levels of a MPDEI and an adenosine uptake inhibitor, which if used alone is not expected to increase muscle blood flow, significantly increase blood flow in the exercised muscle and improves blood flow recovery after a period of ischemia. For example, a combination of cilostazol, with blood concentration ranging from 0.3 to 10 $\mu$M, and dipyridamole, with blood concentration ranging from 0.1 to 10 $\mu$M, produces an optimal profile of platelet aggregation and negligible cardiac side effects. Thus, the combination of at least one MPDEI and at least one adenosine uptake inhibitor, such as cilostazol and dipyridamole, may provide a therapy for conditions such as IC and stroke with improved efficacy but with less cardiac side effects.

The specification is most thoroughly understood in light of the teachings of the references cited within the specification. The embodiments within the specification provide an illustration of embodiments of the invention and should not be construed to limit the scope of the invention. The skilled artisan recognizes that many other embodiments are encompassed by the claimed invention and that it is intended that the specification and examples by considered as exemplary only.

**Reference List**

Cone J, Wang S, Tandon N, Fong M, Sun B, Sakurai K, Yoshitake M, Kambayashi J, and Liu Y (1999) Comparison of the effects of cilostazol and milrinone on intracellular cAMP levels and cellular function in platelets and cardiac cells. J Cardiovasc Pharmacol 34:497-504.

Dobson JGJ and Fenton RA (1998) Cardiac physiology of adenosine, in Cardiovascular Biology of Purines (Burnstock G, Dobson JGJ, Liang BT, and Linden J eds) pp 21-39, Kluwer Academic Publishers, Boston, MA.

Downey JM, Cohen MV, Ytrehus K, and Liu Y (1994) Cellular mechanisms in ischemic preconditioning: the role of adenosine and protein kinase C, in Cellular, Biochemical, and Molecular Aspects of Reperfusion Injury (Das DK ed) pp 82-98, Ann N Y Acad Sci. Vol 723., New York.

George EE, Romano FD, and Dobson JG, Jr. (1991) Adenosine and acetylcholine reduce isoproterenol-induced protein phosphorylation of rat myocytes. J.Mol.Cell Cardiol. 23:749-764.

Gotoh F, Tohgi H, Hirai S, Terashi A, Fukuuchi Y, Otomo E, Shinohara Y, Itoh E, Matsuda T, Sawada T, Yamaguchi T, Nishimaru K, and Ohashi Y (2000) Cilostazol stroke prevention study: A placebo-controlled double-blind trial for secondary prevention of cerebral infarction. J.Stroke and Calebrovasc.Dis. 9:147-157.

Huttemann E, Ukena D, Lenschow V, and Schwabe U (1984) Ra adenosine receptors in human platelets. Characterization by 5'-N-ethylcarboxamido[3H]adenosine binding in relation to adenylate cyclase activity. Naunyn Schmiedebergs Arch.Pharmacol 325:226-233.

Igawa T, Tani T, Chijiwa T, Shiragiku T, Shimidzu S, Kawamura K, Kato S, Unemi F, and Kimura Y (1990) Potentiation of anti-platelet aggregating activity of cilostazol with vascular endothelial cells. Thromb.Res 57:617-623.

Laghi PF, Capecchi PL, Acciavatti A, Petri S, de Lalla A, Cati G, Colafati M, and Di Perri T (1997) Pharmacological preconditioning of ischaemia. Clin Hemorheol.Microcirc. 17:73-84.

Liu Y, Fong M, Cone J, Wang S, Yoshitake M, and Kambayashi J (2000) Inhibition of adenosine uptake and augmentation of ischemia-induced increase of interstitial adenosine by cilostazol, an agent to treat intermittent claudication. J.Cardiovasc.Pharmacol. 36:351-360.

Matsumoto Y, Marukawa K, Okumura H, Adachi T, Tani T, and Kimura Y (1999) Comparative study of antiplatelet drugs in vitro: distinct effects of cAMP-elevating drugs and GPIIb/IIIa antagonists on thrombin-induced platelet responses. Thromb.Res 95:19-29.

Narayan P, Mentzer RM, Jr., and Lasley RD (2000) Phosphatase inhibitor cantharidin blocks adenosine A(1) receptor anti-adrenergic effect in rat cardiac myocytes. Am.J.Physiol Heart Circ.Physiol 278:H1-H7.

Packer M (1992) Treatment of chronic heart failure. Lancet 340:92-95.

Park SW, Lee CW, Kim HS, Lee HJ, Park HK, Hong MK, Kim JJ, and Park SJ (1999) Comparison of cilostazol versus ticlopidine therapy after stent implantation. Am.J.Cardiol. 84:511-514.

Pasini FL, Capecchi PL, and Perri TD (2000) Adenosine and chronic ischemia of the lower limbs. Vasc.Med. 5:243-250.

Sun, B., Le, S., Fong, M., Guertin, M., Liu, Y., Yoshitake, M., Kambayashi, J., and Tandon, N. Interplay between adenosine and cilostazol in antiplatelet activation. Thrombosis and Haemostasis Suppl. 2001.

Thadani U and Roden DM (1998) FDA Panel report: January 1998. Circulation 97:2295-2296.

Tsuchikane E, Fukuhara A, Kobayashi T, Kirino M, Yamasaki K, Izumi M, Otsuji S, Tateyama H, Sakurai M, and Awata N (1999) Impact of cilostazol on restenosis after percutaneous coronary balloon angioplasty. Circulation 100:21-26.

Wang S, Cone J, Fong M, Yoshitake M, Kambayashi J, and Liu Y (2001) Interplay between inhibition of adenosine uptake and phosphodiesterase type 3 on cardiac function by cilostazol, an agent to treat intermittent claudication. J Cardiovasc Pharmacol 38:775-783.

Wang WZ, Anderson G, Maldonado C, and Barker J (1996) Attenuation of vasospasm and capillary no-reflow by ischemic preconditioning in skeletal muscle. Microsurgery. 17:324-329.

Whetzel TP, Stevenson TR, Sharman RB, and Carlsen RC (1997) The effect of ischemic preconditioning on the recovery of skeletal muscle following tourniquet ischemia. Plast.Reconstr.Surg 100:1767-1775.

**Claims**

1. A composition comprising cilostazol and at least one adenosine uptake inhibitor selected from dipyridamole, propentofylline, dilazep, nitrobenzylthioinosine, S-(4-nitrobenzyl)-6-thioguanosine, S-(4-nitrobenzyl)-6-thioinosine, iodohydroxy-nitrobenzylthioinosine, mioflazine, and pharmaceutically acceptable salts thereof.

2. The composition according to claim 1, comprising cilostazol and dipyridamole.

3. A composition consisting essentially of cilostazol and dipyridamole, or salts thereof.

4. The composition of claim 2 or 3, wherein the composition has a cilostazol:dipyridamole weight ratio of about:

   (a) 1:0.7 to about 1:30;

(b) 1:1 to about 1:12; or

(c) 1:1.25 to about 1:12.

5. A pharmaceutical composition comprising the composition of any one of claims 1-4, and one or more pharmaceutically acceptable carriers thereof.

6. The composition according to any one of claims 1-5 for use in therapy.

7. The composition for use in therapy of claim 6, wherein the therapy is:

(a) treatment of peripheral arterial occlusive disease (PAOD) or stroke;
(b) inducing vasodilation and/or blockade of platelet aggregation;
(c) treatment of coronary restenosis; or
(d) reducing smooth muscle proliferation.

8. Use of the composition according to any one of claims 1-5 in the preparation of a medicament for:

(a) treatment of peripheral arterial occlusive disease (PAOD) or stroke;
(b) inducing vasodilation and/or blockade of platelet aggregation;
(c) treatment of coronary restenosis; or
(d) reducing smooth muscle proliferation.

9. The use of claim 8, wherein the composition is administered at about:

(a) 20 mg/day to about 300 mg/day for cilostazol and about 200 mg/day to about 600 mg/day for dipyridamole;
(b) 50 mg/day to about 200 mg/day for cilostazol and about 200 mg/day to about 600 mg/day for dipyridamole;
(c) 50 mg/day to about 160 mg/day for cilostazol and about 200 mg/day to about 600 mg/day for dipyridamole.

10. The composition of any one of claims 1-6 for use according to claim 7(a), or for the use of claim 8(a), wherein the PAOD is intermittent claudication.

**Patentansprüche**

1. Zusammensetzung, die Cilostazol und mindestens einen Adenosin-Aufnahmehemmstoff umfasst, der ausgewählt ist aus Dipyridamol, Propentofyllin, Dilazep, Nitrobenzylthioinosin, S-(4-Nitrobenzyl)-6-thioguanosin, S-(4-Nitrobenzyl)-6-thioinosin, Iodhydroxynitrobenzylthioinosin, Mioflazin und pharmazeutisch verträglichen Salzen davon.

2. Zusammensetzung nach Anspruch 1, die Cilostazol und Dipyridamol umfasst.

3. Zusammensetzung, die im Wesentlichen aus Cilostazol und Dipyridamol oder deren Salzen besteht.

4. Zusammensetzung nach Anspruch 2 oder 3, wobei die Zusammensetzung ein Cilostazol:Dipyridamol-Gewichtsverhältnis aufweist von etwa:

(a) 1:0,7 bis etwa 1:30;
(b) 1:1 bis etwa 1:12; oder
(c) 1:1,25 bis etwa 1:12.

5. Pharmazeutische Zusammensetzung, die eine Zusammensetzung nach einem der Ansprüche 1 - 4 und einen oder mehrere pharmazeutisch verträgliche Träger davon umfasst.

6. Zusammensetzung nach einem der Ansprüche 1 - 5 zur Verwendung in der Therapie.

7. Zusammensetzung zur Verwendung in der Therapie nach Anspruch 6, wobei die Therapie wie folgt ist:

(a) Behandlung von Peripherer Arterieller Verschlusskrankheit (PAOD) oder Schlaganfall;
(b) Induktion von Vasodilatation und/oder Blockade der Blutplättchenaggregation;

(c) Behandlung koronarer Restenose; oder
(d) Verringerung der Proliferation glatter Muskulatur.

8. Verwendung der Zusammensetzung nach einem der Ansprüche 1 - 5 zur Herstellung eines Arzneimittels zur:

(a) Behandlung von Peripherer Arterieller Verschlusskrankheit (PAOD) oder Schlaganfall;
(b) Induktion von Vasodilatation und/oder Blockade der Blutplättchenaggregation;
(c) Behandlung koronarer Restenose; oder
(d) Verringerung der Proliferation glatter Muskulatur.

9. Verwendung nach Anspruch 8, wobei die Zusammensetzung verabreicht wird zu etwa:

(a) 20 mg/Tag bis etwa 300 mg/Tag Cilostazol und etwa 200 mg/Tag bis etwa 600 mg/Tag Dipyridamol;
(b) 50 mg/Tag bis etwa 200 mg/Tag Cilostazol und etwa 200 mg/Tag bis etwa 600 mg/Tag Dipyridamol;
(c) 50 mg/Tag bis etwa 160 mg/Tag Cilostazol und etwa 200 mg/Tag bis etwa 600 mg/Tag Dipyridamol.

10. Zusammensetzung nach einem der Ansprüche 1-6 zur Verwendung nach Anspruch 7(a) oder zur Verwendung nach Anspruch 8(a), wobei die PAOD Claudicatio intermittens ist.

**Revendications**

1. Composition comprenant du cilostazol et au moins un inhibiteur du captage de l'adénosine choisi parmi le dipyridamole, la propentofylline, le dizalep, la nitrobenzylthioinosine, la S-(4-nitrobenzyl)-6-thioguanosine, S-(4-nitrobenzyl)-6-thioinosine, l'iodohydroxy-nitrobenzylthioinosine, la mioflazine et des sels pharmaceutiquement acceptables de ceux-ci.

2. Composition selon la revendication 1, comprenant du cilostazol et du dipyridamole.

3. Composition consistant essentiellement de cilostazol et de dipyridamole, ou de sels de ceux-ci.

4. Composition selon la revendication 2 ou 3, dans laquelle la composition possède un rapport pondéral de cilostazol : dipyridamole d'environ :

(a) 1 : 0,7 à environ 1 : 30 ;
(b) 1 : 1 à environ 1 : 12 ; ou
(c) 1 : 1,25 à environ 1 : 12.

5. Composition pharmaceutique comprenant la composition selon l'une quelconque des revendications 1 - 4, et un ou plusieurs excipients pharmaceutiquement acceptables de ceux-ci.

6. Composition selon l'une quelconque des revendications 1 - 5 destinée à être utilisée en thérapie.

7. Composition destinée à être utilisée en thérapie selon la revendication 6, dans laquelle la thérapie consiste à :

(a) traiter une maladie oblitérante artérielle périphérique (MOAP) ou un accident vasculaire cérébral ;
(b) induire une vasodilatation et/ou le blocage de l'agrégation plaquettaire ;
(c) traiter la resténose coronaire ; ou
(d) réduire la prolifération des muscles lisses.

8. Utilisation de la composition selon l'une quelconque des revendications 1 - 5 dans la préparation d'un médicament consistant à :

(a) traiter une maladie oblitérante artérielle périphérique (MOAP) ou un accident vasculaire cérébral ;
(b) induire une vasodilatation et/ou le blocage de l'agrégation plaquettaire ;
(c) traiter la resténose coronaire ; ou
(d) réduire la prolifération des muscles lisses.

9. Utilisation selon la revendication 8, dans laquelle composition est administrée à environ :

(a) 20 mg par jour à environ 300 mg par jour de cilostazol et à environ 200 mg par jour à environ 600 mg par jour de dipyridamole ;
(b) 50 mg par jour à environ 200 mg par jour de cilostazol et à environ 200 mg par jour à environ 600 mg par jour de dipyridamole ;
(c) 50 mg par jour à environ 160 mg par jour de cilostazol et à environ 200 mg par jour à environ 600 mg par jour de dipyridamole.

10. Composition selon l'une quelconque des revendications 1 - 6 destinée à être utilisée selon la revendication 7(a) ou pour utilisation selon la revendication 8(a), dans laquelle la MOAP est la claudication intermittente.

# Figure 1

**Figure 2**

Figure 3

□Alone ■ + 1 µM Ado ▣ + 1 µM Ado + 30 nM Cil ▨ + 1 µM Ado + 100 nM Cil

**Figure 4**

**Figure 5**

**A**

**B**

**Figure 6**

**Figure 7**

Figure 8

Figure 9

**Figure 10**

A

B

# Figure 11

**Figure 12**

**Figure 13**

## Isolated Rabbit Heart Protocol

15 min        5 min  5 min        10 min        5min

Cilostazol

Cilostazol+Dipyridamole

Dipyridamole

**Figure 14**

**Figure 15**

Muscle stimulation

Ligation  Reperfusion

Surgical Preparation

-60    0    20    40    60    80

Time (min)

Cilostazol+Dipyridamole

● Regional Blood Flow Determination
by Fluorescent Microspheres

**Figure 16**

Gastrocnemius Blood Flow

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **GOODMAN ; GILMAN'S.** The Pharmacological Basis of Therapeutics. 1996, 3-41
- **GENNARO et al.** Remington's Pharmaceutical Sciences. Mack Publishing Company, 1990
- Guide for the Care And Use of Laboratory Animals. National Research Council, 1996
- Manual for Using Fluorescent Microspheres to Measure Organ Perfusion. Fluorescent Microsphere Resource Center
- **CONE J ; WANG S ; TANDON N ; FONG M ; SUN B ; SAKURAI K ; YOSHITAKE M ; KAMBAYASHI J ; LIU Y.** Comparison of the effects of cilostazol and milrinone on intracellular cAMP levels and cellular function in platelets and cardiac cells. *J Cardiovasc Pharmacol,* 1999, vol. 34, 497-504
- Cardiac physiology of adenosine. **DOBSON JGJ ; FENTON RA.** Cardiovascular Biology of Purines. Kluwer Academic Publishers, 1998, 21-39
- Cellular mechanisms in ischemic preconditioning: the role of adenosine and protein kinase C. **DOWNEY JM ; COHEN MV ; YTREHUS K ; LIU Y.** Cellular, Biochemical, and Molecular Aspects of Reperfusion Injury. 1994, 82-98
- *Ann N Y Acad Sci.,* vol. 723
- **GEORGE EE ; ROMANO FD ; DOBSON JG, JR.** Adenosine and acetylcholine reduce isoproterenol-induced protein phosphorylation of rat myocytes. *J.Mol.Cell Cardiol.,* 1991, vol. 23, 749-764
- **GOTOH F ; TOHGI H ; HIRAI S ; TERASHI A ; FUKUUCHI Y ; OTOMO E ; SHINOHARA Y ; ITOH E ; MATSUDA T ; SAWADA T.** Cilostazol stroke prevention study: A placebo-controlled double-blind trial for secondary prevention of cerebral infarction. *J.Stroke and Calebrovasc.Dis.,* 2000, vol. 9, 147-157
- **HUTTEMANN E ; UKENA D ; LENSCHOW V ; SCHWABE U.** Ra adenosine receptors in human platelets. Characterization by 5'-N-ethylcarboxamido[3H]adenosine binding in relation to adenylate cyclase activity. *Naunyn Schmiedebergs Arch.Pharmacol,* 1984, vol. 325, 226-233
- **IGAWA T ; TANI T ; CHIJIWA T ; SHIRAGIKU T ; SHIMIDZU S ; KAWAMURA K ; KATO S ; UNEMI F ; KIMURA Y.** Potentiation of anti-platelet aggregating activity of cilostazol with vascular endothelial cells. *Thromb.Res,* 1990, vol. 57, 617-623

- **LAGHI PF ; CAPECCHI PL ; ACCIAVATTI A ; PETRI S ; DE LALLA A ; CATI G ; COLAFATI M ; DI PERRI T.** Pharmacological preconditioning of ischaemia. *Clin Hemorheol.Microcirc.,* 1997, vol. 17, 73-84
- **LIU Y ; FONG M ; CONE J ; WANG S ; YOSHITAKE M ; KAMBAYASHI J.** Inhibition of adenosine uptake and augmentation of ischemia-induced increase of interstitial adenosine by cilostazol, an agent to treat intermittent claudication. *J.Cardiovasc.Pharmacol.,* 2000, vol. 36, 351-360
- **MATSUMOTO Y ; MARUKAWA K ; OKUMURA H ; ADACHI T ; TANI T ; KIMURA Y.** Comparative study of antiplatelet drugs in vitro: distinct effects of cAMP-elevating drugs and GPIIb/IIIa antagonists on thrombin-induced platelet responses. *Thromb.Res,* 1999, vol. 95, 19-29
- **NARAYAN P ; MENTZER RM, JR. ; LASLEY RD.** Phosphatase inhibitor cantharidin blocks adenosine A(1) receptor anti-adrenergic effect in rat cardiac myocytes. *Am.J.Physiol Heart Circ.Physiol,* 2000, vol. 278, H1-H7
- **PACKER M.** Treatment of chronic heart failure. *Lancet,* 1992, vol. 340, 92-95
- **PARK SW ; LEE CW ; KIM HS ; LEE HJ ; PARK HK ; HONG MK ; KIM JJ ; PARK SJ.** Comparison of cilostazol versus ticlopidine therapy after stent implantation. *Am.J.Cardiol.,* 1999, vol. 84, 511-514
- **PASINI FL ; CAPECCHI PL ; PERRI TD.** Adenosine and chronic ischemia of the lower limbs. *Vasc.Med.,* 2000, vol. 5, 243-250
- **SUN, B. ; LE, S. ; FONG, M. ; GUERTIN, M. ; LIU, Y. ; YOSHITAKE, M. ; KAMBAYASHI, J. ; TANDON, N.** Interplay between adenosine and cilostazol in antiplatelet activation. *Thrombosis and Haemostasis,* 2001
- **THADANI U ; RODEN DM.** FDA Panel report: January 1998. *Circulation,* 1998, vol. 97, 2295-2296
- **TSUCHIKANE E ; FUKUHARA A ; KOBAYASHI T ; KIRINO M ; YAMASAKI K ; IZUMI M ; OTSUJI S ; TATEYAMA H ; SAKURAI M ; AWATA N.** Impact of cilostazol on restenosis after percutaneous coronary balloon angioplasty. *Circulation,* 1999, vol. 100, 21-26

- **WANG S ; CONE J ; FONG M ; YOSHITAKE M ; KAMBAYASHI J ; LIU Y.** Interplay between inhibition of adenosine uptake and phosphodiesterase type 3 on cardiac function by cilostazol, an agent to treat intermittent claudication. *J Cardiovasc Pharmacol,* 2001, vol. 38, 775-783

- **WANG WZ ; ANDERSON G ; MALDONADO C ; BARKER J.** Attenuation of vasospasm and capillary no-reflow by ischemic preconditioning in skeletal muscle. *Microsurgery,* 1996, vol. 17, 324-329
- **WHETZEL TP ; STEVENSON TR ; SHARMAN RB ; CARLSEN RC.** The effect of ischemic preconditioning on the recovery of skeletal muscle following tourniquet ischemia. *Plast. Reconstr. Surg,* 1997, vol. 100, 1767-1775